# EUROPEAN PATENT SPECIFICATION

(11) **EP 1 270 739 B1**
(45) Date of publication and mention of the grant of the patent: **08.03.2006**
(21) Application number: 01114737.8
(22) Date of filing: 22.06.2001
(51) Int. Cl.: C12Q 1/68, C12N 9/16

(54) **Engineered secreted alkaline phosphatase (seap) reporter genes and polypeptides**
Modulierte sekretierte alkalische Phosphatase (SEAP) Reporter Gene und Polypeptide
Gènes reporter de la phosphatase alcaline dont la secretion est modulée par génie génétique et polypeptides

(43) Date of publication of application: 02.01.2003
(73) Proprietor: Aventis Pharma S.A., 92160 Antony (FR)
(72) Inventor: Thuillier, Vincent, Piedmont, CA 94610 (US); Wang, Manping, Fremont, CA 94538 (US); Orsini, Cécile, 75011 Paris (FR)
(74) Representative: Bouvet, Philippe

(56) References cited:
- EP-A- 0 327 960
- BETTAN M. ET AL.,: "secreted human placental alkaline phosphatase as a reporter gne or in vivo gene transfer" ANAL. BIOCHEMISTRY, vol. 271, - 1999 pages 187-189, XP002191938
- GREGORY B ET AL: "A FAST AND SENSITIVE COLORIMETRIC ASSAY FOR IL-6 IN HEPATOMA CELLS BASED ON THE PRODUCTION OF A SECRETED FORM OF ALKALINE PHOSPHATASE (SEAP)9" JOURNAL OF IMMUNOLOGICAL METHODS, ELSEVIER SCIENCE PUBLISHERS B.V.,AMSTERDAM, NL, vol. 170, 29 March 1994 (1994-03-29), pages 47-56, XP000601567 ISSN: 0022-1759
- BARTLETT R J ET AL: "LONG-TERM EXPRESSION OF A FLUORESCENT REPORTER GENE VIA DIRECT INJECTION OF PLASMID VECTOR INTO MOUSE SKELETAL MUSCLE: COMPARISON OF HUMAN CREATINE KINASE AND CMV PROMOTER EXPRESSION LEVELS IN VIVO" CELL TRANSPLANTATION, ELSEVIER SCIENCE, US, vol. 5, no. 3, 1996, pages 411-419, XP002920729 ISSN: 0963-6897

## Description

### Field of the Invention and Introduction

The invention relates to nucleic acids that encode reporter genes capable of being used in long term expression studies and vectors comprising them. Methods for producing vectors for long term expression and the expression systems that incorporate these reporter genes and other transgenes are also included in the invention, as well as the recombinant polypeptides encoded by the reporter genes.

Many gene transfer vectors and systems are intended to express genes for extended periods of time, i.e. over weeks or months. This is especially the case where the transgene encodes a functional protein or therapeutic protein. Methods to evaluate the persistence of expression using these gene transfer vectors usually employ detectable reporter genes. However, the protein products of currently used reporter genes lack one or more of several characteristics, making them inappropriate for studying long term expression in an animal. This invention provides new and useful reporter genes, nucleic acids, expression vectors, polypeptides, and methods of expressing transgenes that are particularly suited for long term expression. Compared to the prior reporter genes, which may express detectable protein up to 20 days or so, the reporter genes of the invention express detectable protein levels for more than about a month, or to at least about 9 months. The expression levels are also stable over this period. Furthermore, the proteins from reporter genes of the invention can be detected in a number of cell types and tissues, where detection using other reporter genes has proven to be difficult.

### Discussion of Related Technology

Reporter genes have been used to analyze the expression of transgenes from various vectors. Reporter genes that have been used in animal models encode exogenous cytoplasmic or secreted proteins, such as bacterial β-galactosidase, insect luciferase, human growth hormone, human erythropoietin, and human secreted alkaline phosphatase (SEAP). These reporter genes often are used for transient expression studies or tissue-specific expression studies. However, the proteins they encode are typically immunogenic. They can also elicit a cytotoxic T-lymphocyte response or a neutralizing antibody response that suppresses detection, leading to inaccurate reporter gene expression data *(see* Tripathy *et al*., Nature Medicine 2:545-50 (1996); and Yang *et al*., Gene Therapy 3:137-44 (1996)).

From these deficiencies alone, one can conclude that the reporter genes currently used were not really designed for and have not been shown to be amenable to longer term expression studies. When longer term expression data is needed, a reporter gene that continues to express, can be detected, avoids the animal's immune response mechanisms, and does not alter cell physiology is important. To date, such a reporter gene has not been adequately provided. In fact, experimental systems have been modified to accommodate the existing reporter genes by, for example, using immuno-deficient animals.

One reporter gene, the SEAP gene noted above, is derived from the native human placental alkaline phosphatase (hPLAP). The amino acid sequence typically used for its reporter gene function differs from the native gene by a deletion of C-terminal residues, which converts the membrane-bound protein into a secreted protein as described by Berger, *et al*. (Gene 66, 1-10 (1988) and in the European patent application EP 327 960. While the SEAP reporter gene has been used in a number of expression systems, the selection of alkaline phosphatase-derived reporter genes often results in relatively short term expression. To this regard, Bettan M. et al. (Anal. Biochemistry, Vol. 271, 1999, pp.187-189) assessed the circulating activity of this secreted human placental alkaline phosphatase protein (SEAP) in plasma after a single injection of a plasmid the coding sequence of the SEAP. It is described that the average SeAP activity peaked at day 7 and slowly decreased to reach 40% of maximal activity at day 28. There is thus a need for a reporter gene which would be a model to study longer term expression.

### Summary of the Invention

The invention encompasses alkaline phosphatase (AP) reporter genes and nucleic acid sequences encoding a mammalian alkaline phosphatase activity. The alkaline phosphatase activity is capable of being detected for at least about one month after being inserted into a cell as a transgene. Preferably, expressing the alkaline phosphatase-encoding sequences of the invention in a cell results in alkaline phosphatase activity that can be detected for more than about 40 days, or more than about 60 days, or more than about 90 days, or more than about 120 days, or more than about 180 days, or more than about 270 days. Typically, but not necessarily, the alkaline phosphatase activity is detected by measuring the protein levels or AP activity of a serum or tissue sample from a mammal containing the cell with the transgene, or measuring the media from a cultured cell containing the transgene. Other methods of detection can be used, including immunochemical assays.

In a preferred embodiment, the mammalian alkaline phosphatase activity consists of, consists essentially of, or comprises a polypeptide amino acid sequence of SEQ ID NO: 1 or SEQ ID NO: 2. A polypeptide or protein consisting essentially of the amino acid sequence of SEQ ID NO: 1 or SEQ ID NO: 2 will contain the amino acid sequence of SEQ ID NO: 1 or SEQ ID NO: 2 and one or more amino acid differences in the sequence that do not change the basic and novel characteristics of the protein. As described, some of the basic and novel characteristics of the mSEAP polypeptide of SEQ ID NO: 2 or the AP activity of SEQ ID NO: 1 are the ability to be expressed and detected in long term expression studies (here long term can mean one of over 30 days, 40 days, 60 days, 90 days, 180 days, or 270 days), the ability to be detected long term in immune-competent mammals, and/or the ability to be expressed long term at stable levels (levels that do not change more than about 5-fold or more than about 10-fold). None of the prior alkaline phosphatase, embryonic alkaline phosphatase, or secreted embryonic alkaline phosphatase proteins or polypeptides possess even one of these novel characteristics. Any isolated or purified cDNA or nucleic acid that encodes a polypeptide of SEQ ID NO: 1 or SEQ ID NO: 2 is included in the invention. Also, the polypeptide of SEQ ID NO: 1 can be joined with an appropriate mammalian or murine signal sequence so the AP activity is secreted from the cell. Any of a variety of signal sequences can be used, including that of SEQ ID NO: 3, 9, 10, 11, or 12. Fusion proteins where the amino acid sequence of SEQ ID NO: 1 or 2 is used are also included in the invention provided they do not include a fusion generating or encoding the exact amino acid sequence of SEQ ID NO: 13. Furthermore, derivatives and mutants of SEQ ID NO: 1 or 2 are included in the invention and specific, non-limiting examples of the mutants are presented. One series of mutants includes polypeptides where about 1 to about 5 amino acids are deleted from the C-terminal end of SEQ ID NO: I or 2. One skilled in the art can use numerous methods to create other derivatives or mutations in SEQ ID NO: 1 or 2 and the degenerate variants encoding SEQ ID NO: 1 or 2 as well as the derivative polypeptides and mutant polypeptides themselves. The derivative polypeptides, and the nucleic acids encoding them, also possess or encode polypeptides that possess one or more of the novel long term expression characteristics listed above, but do not include a polypeptide or SEQ ID NO: 13.

In another aspect, the invention comprises methods for analyzing expression systems and vectors for use in gene transfer experiments and protocols. For example, a gene transfer vector that comprises a reporter gene or nucleic acid sequence of the invention can be introduced into an animal. By detecting reporter gene expression levels, the vector system components and methods used to introduce and prepare the vector for administration can be tested and/or optimized for long term use in expressing a transgene. The methods can be used to select a type of expression vector, specific regulatory or combinations of sequences used in expression vectors, and protocols for administering transgenes to animals. For example, the properties or desirability of selected gene expression regulatory elements (DNA or RNA sequences in *cis*, or protein, DNA, or RNA factors in *trans*) can be analyzed and/or optimized by using the nucleic acids and methods of the invention. Both the selection and analysis processes and the vectors and compositions selected or that result from the processes are specifically included in this invention.

In a preferred embodiment of this aspect, a vector is prepared and introduced into an animal or cell. The vector comprises a reporter gene or nucleic acid of the invention linked to an appropriate promoter or promoter/enhancer so that the reporter gene is expressed. The alkaline phosphatase expression levels are analyzed for at least about one month and optionally compared to controls, such as different vectors comprising the reporter gene. The results of the expression levels for the period of at least about one month are then used to select or confirm that a vector is appropriate or desirable for the administration of a particular transgene. The same methods can be used for periods of at least about 40 days, about 60 days, about 90 days, about 120 days, about 180 days, or about 270 days. The selected vector with particular transgene can then be used for other purposes where long term expression is desired, such as treatment for disease or physiological condition or production of a certain phenotype in an animal. Similarly, administration methods can be analyzed and compared, as for example intramuscular injection, intratumoral injection, intradermal injection, inhalation, or other routes of administration. The type of vector used can also be analyzed, such as naked plasmid, adenoviral vector, adeno-associated virus vector, retroviral vector, and lentiviral vector. Any combination of vector, administration method, and/or compositions used for administration can also be selected or optimized through this method.

In a preferred embodiment, the invention includes a method of expressing a transgene comprising selecting a vector for expressing the transgene, inserting a nucleic acid comprising, consisting essentially of, or consisting of a mSEAP sequence, such as those of SEQ ID NO: 1 or 2, into the vector, whereby the vector causes the expression of or is capable of expressing a mSEAP polypeptide, administering the vector containing the mSEAP sequence to a cell or mammal, detecting the expression of mSEAP polypeptide for a period of about 40 days or more after administering the vector, employing the selected vector without substantial changes in its nucleotide sequence to deliver a transgene product to a cell or animal. The vector without substantial changes in its nucleotide sequence will be the same vector with optional sequence modifications due to the insertion of a different transgene sequence or use of different insertion sites, or sequence modifications that do not result in a substantial change in the expression of the transgene, or sequence modifications that do not substantially effect the function of the vector. As described for this aspect and any aspect of the invention, the transgene can be any number of sequences that encode a polypeptide or protein or any number of sequences that encode a functional transcript. Functional transcripts include, for example, anti-sense nucleic acids, ribozymes, and other nucleic acids intended to act within a cell. The protein or polypeptide-encoding sequences can be those that encode a therapeutic activity or an activity that performs a function in the cell. Numerous transgenes encompassing these activities have been used and can be selected for use with the invention, and the selection of the transgene itself does not limit the scope or practice of the invention.

The vectors that can be used include any cell or composition that allows a nucleic acid to be introduced into a cell. For example, the vectors can be nucleic acids, plasmids, cosmids, recombinant viral vectors, liposomes bearing nucleic acids or recombinant viruses, recombinant or genetically modified cells, and compositions comprising these examples or any combination of these examples.

The invention also provides nucleic acids that encode AP activity and comprise one or more nucleotide substitution, deletion, or addition changes from a nucleotide sequence encoding a polypeptide having the amino acid sequence of SEQ ID NO: 1 or 2, as well as the AP proteins themselves. The AP activity of these proteins allows for the long term expression analysis noted above, for over about one month, or over about 40 days, or about 60 days, or about 90 days, or about 180 days, or about 270 days. Alternatively, the AP activity in cells or animals with introduced nucleic acid sequences is relatively stable over these periods of time, i.e. the levels do not change more than about 5 fold, or more than about 10 fold from the levels detectable after day 10. These nucleic acids and proteins can be produced by any of numerous mutation generating techniques known in the art. The proteins and nucleic acids can be tested for their ability to possess or encode the long term expression activity by the same methods or types of methods described throughout this disclosure.

### Brief Description of the Drawings

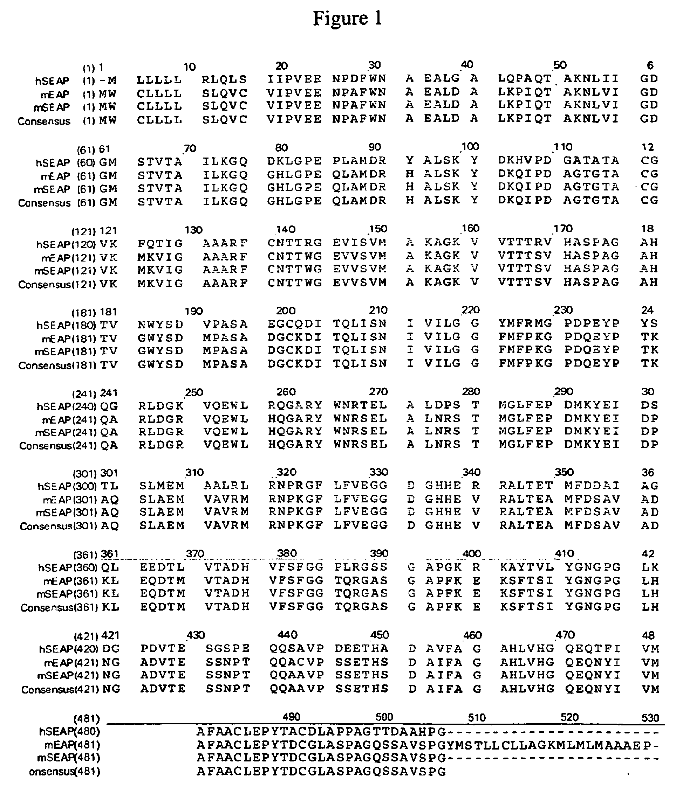

Figure 1 depicts a sequence alignment between hSEAP (translated from GI 2190731 of GenBank), the murine full-length alkaline phosphatase (AP) protein, mEAP (translated from GI 192976 of GenBank), and an engineered mammalian AP of the invention, mSEAP (as translated from the nucleotide sequencing data verified by inventor). The "Consensus" sequence listed at the bottom line is the same sequence as the mSEAP.

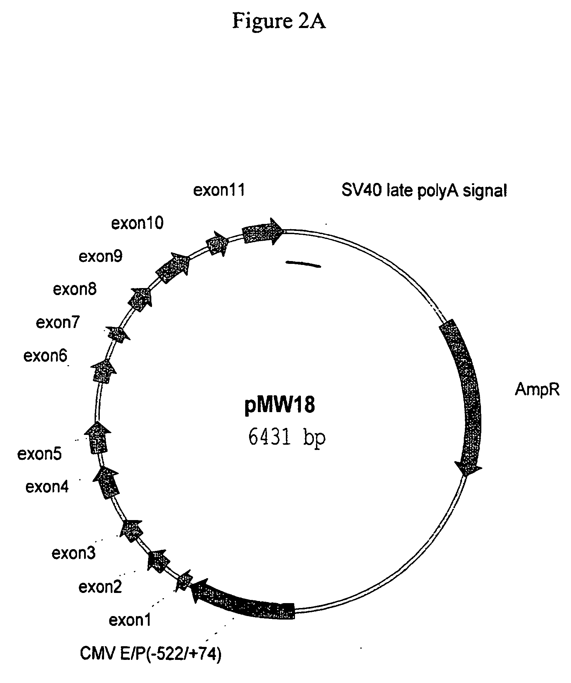

Figure 2A is a plasmid map of pMW18, described in Example 1. It includes the C-terminal truncated form of the EAP coding region.

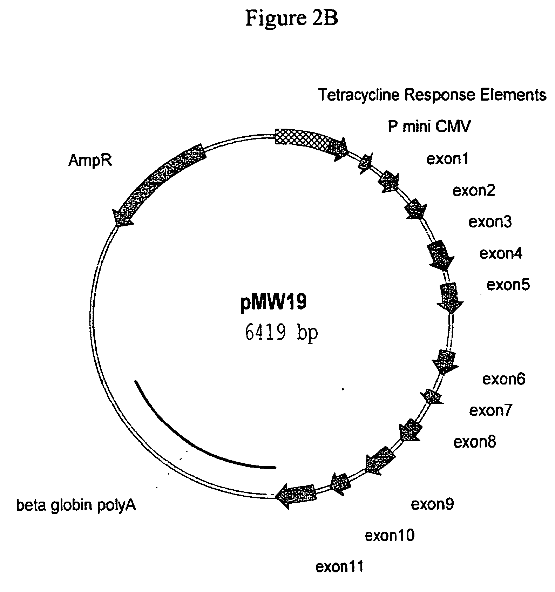

Figure 2B is a plasmid map of pMW19, described in Example 1. It includes the C-terminal truncated form of the EAP coding region.

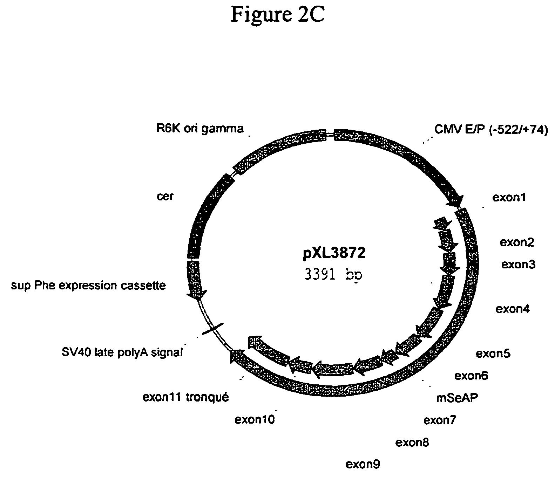

Figure 2C is a plasmid map of pXL3872, described in Example 1. It includes the mSEAP cDNA. Exon 11 has been truncated as described in the examples (listed here as "exon 11 tronqué").

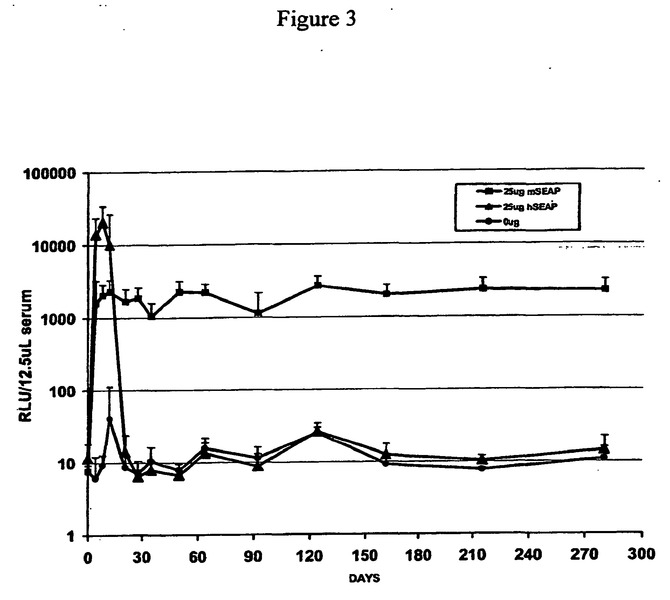

Figure 3 shows the expression levels of alkaline phosphatase activity (AP) following introduction of transgene in Balb/C mice. AP activity is measured by sampling 12.5 µl of serum with the phosphalight kit (Tropix; Bedford, MA). The black boxes represent the expression following introduction of 25µg of plasmid DNA comprising the mSEAP reporter gene of the invention. The black triangles represent the expression levels following the introduction of 25µg of the hSEAP reporter gene known in the art. The black circles represent control where no plasmid DNA was used in the same injection vehicle. The samples are introduced to the mice by intramuscular injection enhanced by electric pulses.

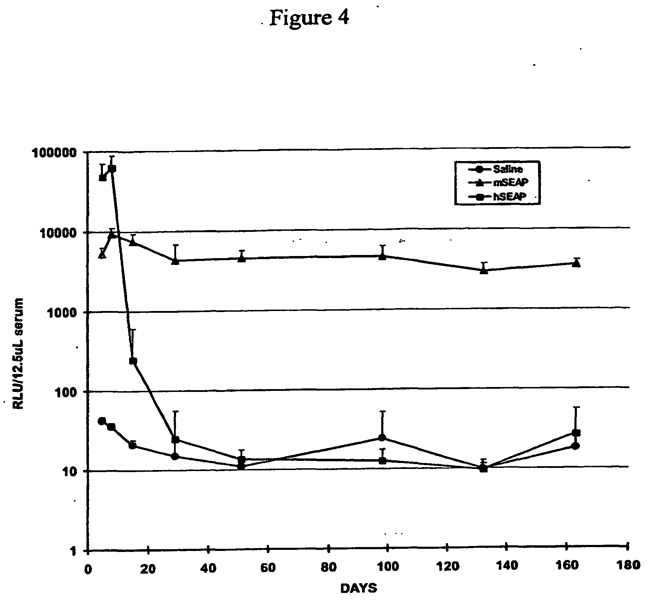

Figure 4 shows the expression levels of alkaline phosphatase activity following introduction of transgene in C57/BL6 mice. The experimental details are the same as in Figure 3. The black boxes represent the expression following introduction of 25µg of plasmid DNA comprising the hSEAP reporter gene known in the art. The black triangles represent the expression levels following the introduction of 25µg of the mSEAP reporter of the invention. The black circles represent control where no plasmid DNA was used in the same vehicle.

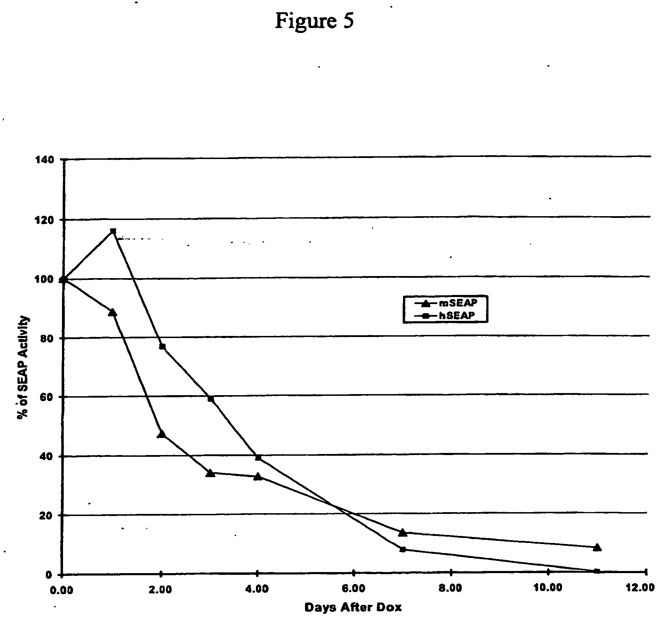

Figure 5 demonstrates the comparable half-life of mSEAP and hSEAP in the bloodstream of Balb/C mice. Two plasmids were co-injected i.m., as per the method of Mir *et al*., PNAS 96:4262-7 (1999): 25µg of a first plasmid encoding the transcription factor tTA, which is active in the absence of doxycycline; and 25µg of a second plasmid encoding either the mSEAP (pMW19) or encoding the hSEAP. Both of the mSEAP and hSEAP sequences are under the control of a tTA-responsive promoter. Alkaline phosphatase activity was assessed 7 days after gene transfer, after which mice were given doxycycline by gavage. Thereafter the mice were given 0.2 mg/ml doxycyline in the drinking water.

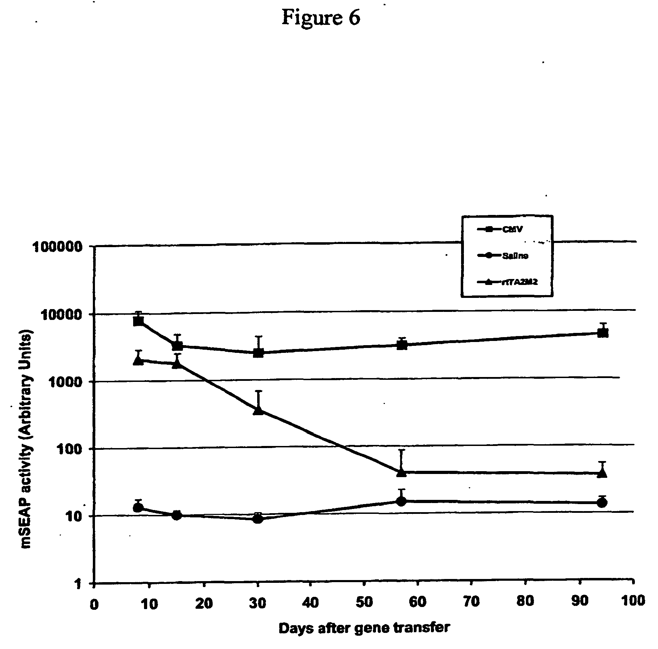

Figure 6 shows the results of a method of analyzing long term expression in a mammal. Balb/C mice are injected i.m. with 25µg plasmid DNA encoding mSEAP under the control of a CMV transcription promoter; or with 25µg plasmid DNA encoding mSEAP under the control of rtTA2M2 in combination with 25µg plasmid DNA encoding rtTA2M2; or with saline as control. *See* Urlinger *et al., PNAS* 97: 7963-68 (2000). Gene transfer was enhanced by electric pulses according to Mir *et al*. (1999), noted above. Mice were given 0.2 mg/ml doxycycline in the drinking water. The AP activity is assessed at the indicated time points.

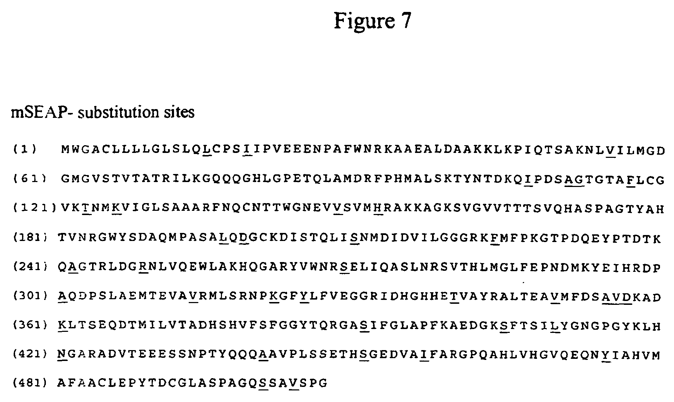

Figure 7 shows the amino acid sequence of mSEAP, with underlined amino acids that can be replaced by conservative amino acid substitutions to create mutants or derivative polypeptides of mSEAP.

### Detailed Description of Exemplary Embodiments

In making and using aspects and embodiments of this invention, one skilled in the art may employ conventional molecular biology, virology, microbiology, and recombinant DNA techniques. Exemplary techniques are explained fully in the literature and are well known in the art. For example, one may rely on the following general texts to make and use the invention: Sambrook *et al., Molecular Cloning: A Laboratory Manual,* Second Edition (1989) Cold Spring Harbor Laboratory Press, Cold Spring Harbor, New York and Sambrook *et al*. Third Edition (2001); *DNA Cloning: A Practical Approach,* Volumes I and II (D.N. Glover ed. 1985); *Oligonucleotide Synthesis* (M.J. Gaited. 1984); *Nucleic Acid Hybridization* (B.D. Hames & S.J. Higgins eds. (1985)); *Transcription And Translation* Hames & Higgins, eds. (1984); *Animal Cell Culture* (RI. Freshney, ed. (1986)); *Immobilized Cells And Enzymes* (IRL Press, (1986)); B. Perbal, *A Practical Guide To Molecular Cloning* (1984); F.M. Ausubel et al. (eds.), *Current Protocols in Molecular Biology,* John Wiley & Sons, Inc. (2001), Coligan et al. (eds.), *Current Protocols in Immunology,* John Wiley & Sons, Inc. (2001); Dracopdi et al., *Current Protocols in Human Genetics*, John Wiley & Sons, Inc. (2001), W. Paul et al. (eds.) *Fundamental Immunology,* Raven Press; E.J. Murray *et al*. (ed.) *Methods in Molecular Biology: Gene Transfer and Expression Protocols,* The Humana Press Inc. (1991); and J.E. Celis *et al., Cell Biology: A Laboratory Handbook*, Academic Press (1994).

As used herein, a "vector" means any nucleic acid or nucleic acid-bearing particle, cell, or organism capable of being used to transfer a nucleic acid into a host cell. The term "vector" includes both viral and nonviral products and means for introducing the nucleic acid into a cell. A "vector" can be used *in vitro, ex vivo,* or *in vivo.* Non-viral vectors include plasmids, cosmids, and can comprise liposomes, electrically charged lipids (cytofectins), DNA-protein complexes, and biopolymers, for example. Viral vectors include retroviruses, lentiviruses, adeno-associated virus, pox viruses, baculovirus, reoviruses, vaccinia viruses, herpes simplex viruses, Epstein-Barr viruses, and adenovirus vectors, for example.

A "nucleic acid" is a polymeric compound comprised of covalently linked nucleotides, from whatever source. Nucleic acid includes polyribonucleic acid (RNA) and polydeoxyribonucleic acid (DNA), both of which may be single-stranded or double-stranded. DNA includes cDNA, genomic DNA, synthetic DNA, and semi-synthetic DNA. The term "nucleic acid" also captures sequences that include any of the known base analogues of DNA and RNA.

Percent "identity" between two nucleic acids or two polypeptide molecules refers to the percent defined by a comparison using a basic blastn or blastp search at the default setting (*see, for example,* NCBI BLAST home page: http://www.ncbi.nlm.nih.gov/BLAST/). "Homology" can be determined by a direct comparison of the sequence information between two polypeptide molecules by aligning the sequence information and using readily available computer programs. Alternatively, homology can be determined by hybridization of polynucleotides under conditions allowing for the formation of stable duplexes between homologous regions and determining of identifying double-stranded nucleic acid.

One or more amino acid residues within a sequence can be substituted by another amino acid of a similar polarity, which acts as a functional equivalent when the substitution results in no significant change in activity in at least one selected biological activity or function. A derivative polypeptide will be a functional equivalent of a given amino acid sequence. One or more substitutions at the positions indicated (by underlining) in Figure 7 can be made in SEQ ID NO: 1 or 2 to produce a functionally equivalent, derivative polypeptide. Truncations from the C-terminal end of SEQ ID NO: 1 or 2 can also be made to produce a functionally equivalent, derivative polypeptide. Conservative substitutions for an amino acid within a sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar, neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Alterations of an amino acid with another amino acid from the same class will not substantially effect activity, apparent molecular weight as determined by polyacrylamide gel electrophoresis, or significantly affect the isoelectric point.

"Isolated," when referring to a nucleic acid, gene, or vector, means that the indicated molecule is present in the substantial absence of other biological macromolecules of the same type. Thus, an "isolated nucleic acid molecule that encodes a particular polypeptide" refers to a nucleic acid molecule substantially free of other nucleic acid molecules that do not encode the particular polypeptide. However, the preparation or sample containing the molecule may include other components of different types. In addition, "isolated from" a particular molecule may also mean that a particular molecule is substantially absent from a preparation or sample.

As used herein, a "reporter gene" can refer to a nucleic acid that encodes a detectable polypeptide or protein, such as AP, or can refer to the protein product of that expressed nucleic acid. Thus, the mSEAP reporter gene and protein can mean the genomic DNA encoding EAP modified to become or encode a mSEAP polypeptide, or a modified cDNA encoding a mSEAP, or the mSEAP protein itself (the mSEAP reporter gene product). The novel mSEAP reporter gene and protein of the invention specifically includes SEQ ID NO: 1, SEQ ID NO: 1 in combination with a mammalian signal sequence at the N-terminal end, SEQ ID NO: 1 in combination with a murine signal sequence at the N-terminal end, SEQ ID NO: 2, any of the above with about 1 to about 5 amino acids deleted from the C-terminal end, and any derivative or mutant of SEQ ID NO: 1 or 2 that has not previously been disclosed to the public or in an application for patent, as well as the nucleic acids that encode these sequences. As used herein, a derivative or mutant can be a sequence with about 80% identity, about 90% identity, or about 95% identity with SEQ ID NO: 1 or 2 using blastp or blastn at the default settings, as well as nucleotide sequences encoding them. A derivative polypeptide can also possess the long term expression characteristics of being detectable over at least about 40 days after insertion into a cell. The mSEAP polypeptides or reporter gene products of the invention do not include a polypeptide with the exact amino acid sequence of SEQ ID NO: 13.

Numerous gene transfer methods and techniques can be used in conjunction with the invention beyond those specifically described. Many of the references listed can be used in selecting an appropriate gene transfer technique, composition, or delivery method. Reporter genes have been used in a number of mammalian test subjects, including mouse, rabbit, cat, dog, primates, and humans. One of skill in the art is familiar with the techniques and methods appropriate for these mammalian test subjects. *See, for example,* Rosenberg *et al*., *New Eng. J. Med.* 323: 570-78 (1990); Cavazzana-Calvo *et al., Science* 288: 669-72 (2000); Dobson *et al., Brit. Med. J*. 320: 1225 (2000); Buckley *et al., Nature Med.* 6: 623-24 (2000); and the general texts and references listed above.

### Some Experimentally Determined Properties and Characteristics of mSEAP

We have engineered the mouse embryonic alkaline phosphatase (mEAP) gene to be amenable for long term expression studies and the analysis of gene transfer methods and vectors. In so doing, we have addressed or solved several problems relating to the use of reporter genes in long term expression analysis. For example, the background AP activity in some animal tissue is quite high, for example plasma from mice. Heating plasma samples at 65°C for 5 to 30 minutes can reduce the background from tissue non-specific alkaline phosphatase (TNAP) activity. Furthermore, inhibitors, such as L-homoarginine, can be used to reduce background TNAP activity. Pretreatment with heat and/or inhibitors is commonly used with AP activity assays. (Cullen and Malim, Meth. Enzymol. 216:362-368 (1992)). Until now, it was not known whether an engineered form of mEAP would be thermostable enough and resistant enough to TNAP inhibitors for use as a reporter gene. Furthermore, it was not known if a reporter gene, and particularly an AP reporter gene, could be expressed and detected over long periods of time. Since the mEAP protein is naturally expressed in embryonic cells in mice, the expression of the engineered mSEAP protein may be subject to an endogenous or physiological regulation that shuts it off, rendering it useless as a reporter gene.

The mSEAP reporter gene sequences, nucleic acids, and proteins of the invention are the first that can be expressed and detected over several months in the classical laboratory, immuno-competent mammal after somatic gene transfer. The same vectors and nucleic acids can be used in a number of mammals, including, but not limited to, mouse, rat, pig, rabbit, goat, cow, sheep, macaque, cynomolgus macaque, and human. The freedom to use immuno-competent animals or to avoid treatments that inhibit the immune response reduces the complexity of the protocol, reduces the added risk to the animal, and reduces the cost of gene transfer experiments. Thus, the invention provides at least an economic advantage compared to other available methods for analyzing long term expression. The invention also provides the advantage of analyzing expression in a physiologically normal patient or animal. In addition, AP activity or the presence of the mSEAP protein can be assayed by several simple techniques, including those employing chemiluminescent, fluorescent, or other detectable substrates and assays.

We have demonstrated the use and advantages of the invention in an intramuscular gene transfer experiment using immuno-competent mice. For example, Figure 3 shows that the level of expression of mSEAP reporter genes of the invention are stable over more than 250 days. In contrast, other reporter genes show a level of expression that drops after 21 days and the levels vary exponentially within the period after the initial ability to detect the activity. The result is consistent in two strains of mice with differing genetic backgrounds (Balb/C and C57/BL6). The lower activity of mSEAP is due to the presence of alkaline phosphatase inhibitors present in the detection kit (PhosphaLight, Tropix; Bedford, MA), which affect mSEAP activity but not hSEAP activity (data not shown).

We have also demonstrated that an immune response against hSEAP is the probable cause of the drop in hSEAP activity because we detected anti-hSEAP antibodies in mice injected with plasmid encoding hSEAP *(see* Table 1).

**Table 1**

| | 1 | 2 | 3 | 4 | 5 | 6 | 7 | 8 |
|---|---|---|---|---|---|---|---|---|
| | 64ng/ml mAb | Neg control: | CMV-hSEA | CMV-hSEA | CMV-hSEA | CMV-hSEA | CMV-hSEAP | positive control: |
| | (Sigma) | serum | P #1 | P #2 | P #3 | P #4 | #5 | mAb in neg serum |
| undiluted | 0.486 | 0.036 | 0.473 | 0.52 | 0.488 | 0.4 | 0.518 | 0.546 |
| 1:2 | 0.487 | 0.036 | 0.539 | 0.525 | 0.532 | 0.358 | 0.576 | 0.439 |
| 1:4 | 0.424 | 0.041 | 0.551 | 0.497 0.53 | 0.53 | 0.344 | 0.512 | 0.341 |
| 1:8 | 0.282 | 0.033 | 0.469 | 0.434 | 0.484 | 0.264 | 0.395 | 0.209 |
| 1:16 | 0.169 | 0.022 | 0.328 | 0.369 | 0.4 | 0.199 | 0.371 | 0.123 |
| 1:32 | 0.091 | 0.018 | 0.275 | 0.3 | 0.309 | 0.118 | 0.262 | 0.071 |
| 1:64 | 0.047 | 0.01 | 0.167 | 0.217 | 0.215 | 0.077 | 0.167 | 0.042 |
| 0 | 0.007 | 0.005 | 0.006 | 0.006 | 0.005 | 0.007 | 0.007 | 0.009 |

In Table 1, the detection by ELISA of anti-hSEAP antibodies in mice serum after i.m. electrotransfer of 25 µg plasmid containing DNA encoding hSEAP driven by the cytomegalovirus immediate early gene promoter (CMV) is shown. Microplates are coated with purified hPLAP (SIGMA, St. Louis, MO) and incubated with serum of mice injected with saline (neg serum: row #2) or hSEAP encoding plasmid (CMV-hSEAP, rows #3-7), or with anti-hPLAP monoclonal antibody diluted in PBS (row #1) or serum of non injected mice (row #8). Anti-hSEAP antibodies are detected by an anti-mouse antibody-HRP conjugate. After reaction with TMB the absorbance is read at 450 nm. Comparing the results of the negative control serum, row #2, with those of row # 3-7 shows that for each of the samples where hSEAP plasmid is injected and then expressed (row # 3-7) there are detectable levels of anti-hSEAP antibodies. This indicates that using the hSEAP reporter gene deleteriously results in the production of antibodies, confounding the results of expression assays and inappropriately inducing an immune response in the animal tested.

Furthermore, we have also demonstrated that the stable levels of mSEAP activity shown in Figure 3 is not due to its stability in the bloodstream but rather to its sustained and persistent synthesis or expression by the cells bearing the transgene. In Figure 5, we measure the decay of mSEAP and hSEAP activity after shutting down transcription. To accomplish this, we co-injected mice with two plasmids: one plasmid encoding the transcription factor tTA, which is active in the absence of doxycycline; and the second plasmid encoding mSEAP (pMW19) or hSEAP under the control of a tTA-responsive promoter (*see, for example,* Urlinger, *et al*., PNAS 97: 7963-68 (2000)). The mice are given doxycyline 7 days after gene transfer and the alkaline phosphatase activity assessed thereafter.

The results in Figure 5 show that the half-life of mSEAP activity is less than 2 days and that the half-life of hSEAP activity is less than 4 days. These numbers take into account the half-life of the reporter protein, of its mRNA, and the decay of the transcription rate upon doxycycline administration. Hence, the sustained mSEAP activity shown in Figure 3 cannot be explained by a prolonged half-life ofmSEAP in the bloodstream.

The utility of this invention is further demonstrated by the experiment presented in Figure 6. We assessed the impact of the chimeric transcription factor rtTA2M2 (*see, for example*, Urlinger, *et al*., PNAS 97: 7963-68 (2000)) on the duration of mSEAP expression. In this experiment, mice are injected with a plasmid expressing mSEAP under the control of a constitutive transcription promoter (CMV) or with a plasmid expressing mSEAP under the control of a rtTA2M2-responsive promoter in combination with a rtTA2M2-encoding plasmid. Animals are given doxycycline (a water-soluble derivative of tetracycline) in the drinking water because rtTA2M2 is activated by this molecule. This experiment demonstrates clearly that rtTA2M2 is not capable of sustaining high levels of expression of mSEAP for more than 15 days. This may be due to a cytotoxic immune response against the cells expressing rtTA2M2 and mSEAP (rtTA2M2 is a fusion protein made of domains of bacterial and viral origins). In contrast, the CMV-driven expression of mSEAP results in stable levels of expression. Thus, the method of expressing a transgene or the method of analyzing long term expression of a transgene provided by this invention allows one to select appropriate regulatory elements and vectors for long term expression applications. Numerous regulatory elements exist in the art and can be selected for use and analysis. The invention is not limited to the use of any particular regulatory element or those specifically exemplified here.

The results discussed above and in the following specific examples are merely representative of the scope of the invention and content of this disclosure. One skilled in the art can use the information here to devise, produce, and use additional embodiments of the invention. Thus, the examples given here should not be taken as a limitation on the scope or extent of the invention.

### Example 1. Production of Plasmids Comprising AP Activity-encoding Nucleic Acids

The nucleic acid encoding the mSEAP reporter gene or polypeptide of the invention can be derived from mouse genomic DNA, synthesized from a GenBank sequence, or derived from one of the plasmids containing an *EAP* gene (for example, pSVT7-*EAP* from Narisawa *et al*., Development 116:159-165 (1992), Manes *et al*., Genomics 8: 541-554 (1990); or those of Hahnel *et al*., Development 110: 555-564 (1990); Bao *et al*., Gynocologic Oncology 78:373-379 (2000); Berger *et al*., Gene 66:1-10 (1988)). From either the cloned gene sequence or the genomic sequence, appropriate PCR oligos are prepared that result in the deletion of about 22 codons from those immediately preceding the stop codon. Functionally, the deletion of the C-terminal codons removes the membrane anchoring activity possessed by the native C-tenninal amino acids. Accordingly, any C-terminal truncation resulting in a polypeptide that functionally lacks a membrane anchoring activity can be used and is included in this invention.

A stop codon is added or supplied to the end of the coding region. For example, a mouse genomic fragment encoding EAP can be amplified by PCR with oligonucleotides A and B below to generate the mSEAP of SEQ ID NO: 2. Alternatively, oligos A and C can be used to generate the full-length EAP encoding fragment. The full-length fragment can be used to create mutations or as a control.

In one method, the PCR fragments are cut by *Hind*III and *Xba*I and inserted between the *Hind*III and *Avr*II sites of pXL3443 (a pBKSII-derived plasmid containing the CMV I/E promoter and the SV40 late poly(A) signal separated by a multiple cloning site) to generate pMW12 and pMW18 *(see* Figure 2A). The same insert can be used between the *Hind*III and *Xba*I sites of pTRE2 (Clontech) to generate pMW19 *(see* Figure 2B). Plasmid pMW12 harbors the native *EAP* gene and pMW18 the genomic clone with a truncation at the C-terminal end of the EAP coding region, both under the control of the CMV I/E promoter (from about -522 to about +74 from the start), and the SV40 late poly(A) signal. Plasmid pMW19 directs the transcription of the truncated murine EAP gene from a CMV I/E minimal promoter (from about -51 to about +70 from the start) under the control of a tetracycline response element (*see,* for example, Gossen and Bujard, PNAS 89: 5547-5551 (1992); (Urlinger, *et al*., PNAS 97: 7963-68 (2000)), with a β-globin poly(A) signal.

A cDNA encoding mSEAP of SEQ ID NO: 2 can be obtained as follows. Murine C2C12 myoblasts are transiently transfected with pMW18 complexed to LipofectAMINE (Life Technologies, Gaithersburg, MD) according to the manufacturer's protocol. PolyA⁺ RNA is extracted from transfected cells with a commercial kit (Dynal dynabeads mRNA direct kit). PolyA⁺ RNA is reverse-transcribed and amplified by PCR (RT-PCR kit Promega, Madison, WI) with oligonucleotides C9415 and C9416, shown below.

Oligos C9415 and C9416 are designed to amplify the mSEAP nucleic acid flanked at the 5' end by a *Hind*III site, and flanked at the 3' end by an *Xba*I site. The product of the RT-PCR reaction is ligated in pGEMT-easy (Promega, Madison, WI) and transformed into *E. coli* DH5α. The resulting plasmid contains the mSEAP cDNA surrounded by a multiple cloning site. A plasmid pCOR (*see* Soubrier *et al*., Gene Therapy 6: 1482-88 (1999)) can then be used to construct a plasmid pXL3872 (Figure 2C) containing the mSEAP nucleic acid under the control of the CMV I/E promoter (-522/+74) and the SV40 late poly(A) signal using the *Hind*III to *Xba*I sites of pXL3856 (a pCOR plasmid containing the CMV I/E promoter (-522/+74) and the SV40 late poly(A) signal). The expression cassette region of plasmid pXL3872 (Figure 2C) is verified by sequencing.

Plasmids encoding other AP sequences are constructed in like manner for control AP proteins, for other mSEAP polypeptides of the invention, or derivative mSEAP polypeptides of the invention. For example, pXL3402 is a pCOR (Soubrier *et al*., 1999) plasmid with the SEAP cDNA under the control of the CMV I/E promoter (-522/+74) and the SV40 late poly(A) signal. Other plasmids put the SEAP under the control of the tetracycline responsive elements (TRE) and the SV40 late poly(A) signal. In another example, the *Pst*I*-Spe*I fragment encoding luciferase in pBi-L (Baron *et al*., Nucleic Acids Res. 23:3605-06 (1995)) can be replaced by nucleic acid encoding an AP activity flanked by the *Pst*I and *Spe*I sites. A PCR amplified fragment from pSEAP2-basic (Clontech) can be used. Also, a plasmid with an AP activity under the control of the CMV I/E promoter and the SV40 late poly(A) signal can be constructed by replacing the *Sac*I*-Pvu*II fragment of pBi-L (TRE) by the *Sac*I*-Stu*I fragment of pXL3031 (CMV I/E promoter, Soubrier *et al*., 1999) and subsequently replacing the *luciferase* ORF by the *SEAP* ORF.

The plasmids can be prepared for gene transfer administration by purification with the endo-free Mega-Prep kit (Qiagen). Preferably, the endotoxin level detected in the samples is less than 20 EU per mg of DNA. Other methods and techniques for purifying vectors and nucleic acids for administration to a mammal are known in the art and can be used.

### Example 2: Transfection of Cultured Cells

C2C12 (ATCC: CRL1772), and HEK293 (ATCC: CRL1573) cells are seeded in 24-well plates (7.5 x 10⁴ cells per well) and grown for 24 h in DMEM supplemented with 10% FCS. Cells are then washed in DMEM without serum and transfected in triplicate by adding to the cells 0.5 ml of OptiMEM mixed with various quantities of AP-encoding plasmid, supplemented to 500 ng with a carrier plasmid and LipofectAMINE (2 µl for C2C12, 3 µl for HEK293). Five hours later, the medium containing the DNA and the LipofectAMINE is replaced by 1 ml of DMEM supplemented with FCS (2% for C2C12, 10% for HEK293). Aliquots of the culture medium are collected 2 days post-transfection and frozen at -70°C for storage. The cells are rinsed twice with PBS, incubated with 100 µl of 0.2% Triton X-100, 50 mM Tris-HCl pH 7.4, 150 mM NaCl, detached from the plate with a scraper and homogenized by repeated pipetting. The lysate is centrifuged 2 minutes at maximum speed in an eppendorf tabletop centrifuge, and the supernatant stored at -70°C.

### Example 3: Intramuscular Gene Transfer

Eight-week-old female Balb/C or C57BL/6 mice (Charles River Laboratories) are anesthetized by intraperitoneal injection of 200 µl ketamine (8.66 mg/ml) mixed with xylazine (0.31 mg/ml) in 150 mM NaCl. The hind legs are shaved. Twenty-five microliters of a nucleic acid vector containing solution in 150 mM NaCl are injected in the tibialis cranialis muscle. Thirty seconds after injection, transcutaneous electric pulses can be applied though stainless steel parallel electrodes connected to an Electro Square Porator, Model T820 (BTX, San Diego, CA), and a TDS 210 oscilloscope (Tektronix, Oregon). If used, the plate electrodes are placed on each side of the leg (plate separation distance 4 mm) and 8 square wave pulses (80 volts, 20 ms each pulse, 1 pulse per second) are applied (Mir *et al*., 1999).

At different time points relative to DNA injection, 50 µl blood samples are collected from the saphenous vein into heparinized capillary tubes (Hem *et al*., Lab. Anim. 32:364-68 (1998)). Samples are spun 20 minutes at 2000 rpm in a clinical centrifuge and plasma collected and frozen at -70°C for storage.

### Example 4: Reporter Gene Expression Analysis

### Exemplary Enzymatic Assay

Detection of the AP activity can be carried out with the Phospha Light kit (Tropix, Perkin-Elmer), following the manufacturer's instructions or by using the modifications of Cullen and Malim, Meth. Enzymol. 216: 362-8 (1992)). Fifteen (15) µl of plasma or cell culture medium or cell extract are diluted into 45 µl of 1X dilution buffer (50 mM Tris-HCl pH 7.4; 150 mM NaCl), heated at 65°C for 5 minutes, then cooled down to 4°C in a PCR block thermocycler. Fifty (50) µl of diluted sample are incubated 5 minutes at room temperature with 50 µl of assay buffer (1M diethanolamine pH 10.5-11, 1mM MgCl2, 10 mM L-homoarginine) before adding 50 µl of reaction buffer (CSPD substrate with Emerald enhancer). The reaction is then incubated 20 minutes at room temperature before chemiluminescence measurement (10 seconds per well) in a MLX microtiter plate luminometer (Dynex).

### Exemplary ELISA Assay

Detection of antibodies directed against AP, for example the hSEAP in Table 1, can be performed using an ELISA assay. However, similar assays for other AP proteins, such as those of the invention, can also be used. Wells of a PVC microtiter plate are coated with 50 µl of a 0.5 µg/ml solution of human placental alkaline phosphatase (PLAP, Sigma) in 0.2 M Na₂CO₃/NaHCO₃ pH 9.5 and incubated overnight at 4°C. The plate is washed 3 times with 250 µl of 0.1 M phosphate buffer pH 7.5, 0.1 M NaCl (PBS), 0.05% Tween 20 (w/v). The remaining sites for protein binding on the PVC plate are saturated by incubation with PBS, 2% BSA (w/v) for 2 hours at room temperature. Fifty microliters of test plasma or positive or negative controls are added per well and incubated 2 hours at room temperature. The negative control can be the plasma of untreated Balb/c mouse. The positive control is obtained by spiking the plasma of a negative control mouse with 64 ng/ml of a mouse monoclonal antibody to PLAP (clone 8B6, Sigma Immunochemicals). Plasma samples are tested at dilutions 1:1 to 1:64 (two-fold serial dilutions in PBS). Samples are then incubated 1 hour at room temperature with a goat anti-rabbit IgG conjugated to horseradish peroxidase (Bio-Rad) in PBS, 2% BSA (w/v), 0.05% Tween 20, and washed 3 times with 250 µl of PBS, 0.05% Tween 20. The samples are reacted with 100 µl 3,3',5,5'-tetramethylbenzidine (USB, Ohio) for 15 minutes at room temperature, and the reaction stopped by adding 50 µl of 0.5 N H₂SO₄. The absorbance is read at 450 nm.

### Example 5: Long Term Expression Analysis

Mice are injected i.m. with 25µg plasmid DNA encoding hSEAP, mSEAP of the invention, or saline control. Gene transfer is enhanced by electric pulses according to Mir, *et al*., PNAS 96:4262-7 (1999). Blood samples are collected in heparinized capillaries at the indicated time points by saphenous vein puncture, spun 20 minutes at 2000 rpm in a clinical centrifuge, and 12.5 µl of serum assessed for alkaline phosphatase activity according to the instructions in the phosphalight kit (Tropix), except that diluted plasma samples are heated 5 minutes at 65°C. Results for exemplary experiments of this type are shown in Figure 3. The mSEAP levels, at the top of the chart, are detected in the first sample after gene transfer and remain consistently high throughout the entire sampling period (over 9 months). In contrast, the hSEAP reporter gene can be detected at levels above control for only about 20 days, and the levels vary greatly. The levels and relative errors reported indicate that the inventive mSEAP reporter genes can be used for extremely long expression studies and that the levels of expression are consistent and reproducible.

### Example 6: Preparation of mSEAP-encoding Nucleic Acids and Derivative Polmeptides; Analyzing Expression Vectors

The nucleic acids derived from the mEAP gene noted in Example 1 can be used as the starting point to generate conservative amino acid substitution mutants of the mSEAP proteins of the invention. The mSEAP encoding sequence is PCR amplified with an oligo that incorporates one of more nucleotide changes that result in amino acid substitutions. For example, the method described in Ausubel *et al*. Current Protocols in Molecular Biology (chapter 3 and unit 3.17, and chapter 8 and unit 8.5, in particular) can be adapted. The amino acids noted by the underlining in Figure 7 represent sites for conservative amino acid changes. Positions 358, 357, or 356 can be modified to encode an isoleucine in place of a valine, and/or a glutamic in place of an aspartic, and/or an arginine in place of a lysine. Once inserted into the sequence, the substitution mutant sequence can be incorporated into a plasmid as in Example 1. The mutant mSEAP protein can then be expressed as in Example 5 and tested to confirm long term expression and/or the ability to be used in immuno-competent mammals. Any derivative polypeptide possessing the long term expression characteristics noted above and throughout this disclosure is included in the invention. Testing for these characteristics can be performed as described here.

Exemplary derivative polypeptides can be made using the same techniques used in Example 1 to generate mSEAP nucleic acids. The 3' primer "B", SEQ ID NO: 5, encodes a stop codon resulting in a 22 amino acid truncation in the incorporated PCR amplified product with the A primer, SEQ ID NO: 4.

Selecting a new 3' primer by sliding the stop codon further into the coding sequence results in amplified products that contain fewer codons. Thus, truncated products are produced from the SEAP or EAP cDNAs or genomic clones nucleotides that correspond to 27 amino acid truncations from the murine EAP gene noted in Manes *et al*., Genomics 8:541-554 (1990). Truncations of about 22 to about 27 amino acids from the mEAP are made in this way. The resulting, amplified DNA is then incorporated into a mammalian expression vector, administered to an immuno-competent mouse, and the long term expression characteristics of the AP-activity is tested by the chemiluminescent assay (Tropix; Bedford, MA). The expression vector can be viral, such as adenovirus or adeno-associated virus, or plasmid vectors. By testing the AP-activity using particular expression vectors or regulatory sequences, an appropriate vector can be selected or optimized for transgene expression. Ligand-dependent regulatory sequences can also be tested for their ability to control expression over long periods of time (*see, for example*, Abruzzese, *et al*., Hum. Gene Ther. 10:1499-1507 (1999); Urlinger *et al., PNAS* 97: 7963-68 (200)), such as the 40 day, 60 day, 90 day, 120 day, 180 day, or 270 day periods possible through the use of the invention.

## Claims

1. An isolated nucleic acid comprising a nucleotide sequence that encodes a mammalian alkaline phosphatase activity with the amino acid sequences of SEQ ID NO: 1 or 2, wherein the nucleic acid does not encode a polypeptide with the same sequence of amino acids listed in SEQ ID NO: 13.

2. An expression vector comprising the nucleic acid of claim 1.

3. A viral vector comprising the nucleic acid of claim 1.

4. A plasmid vector comprising the nucleic acid of claim 1.

5. A mammalian cell into which a nucleic acid of claim 1 or a vector of one of claims 2-4 has been introduced in vitro.

6. The cell of claim 5, that is a mouse cell.

7. The cell of claim 5 that is a human cell.

8. A nucleic acid having a sequence that encodes a mammalian alkaline phosphatase activity-possessing polypeptide, wherein the polypeptide is capable of being expressed and detected in a mammal for a period of about 40 days, and wherein the nucleic acid encodes a polypeptide with the amino acid sequence of SEQ ID NO: 1 or 2 but not that of SEQ ID NO: 13.

9. An expression vector comprising the nucleic acid of claim 8.

10. A viral vector comprising the nucleic acid of claim 8.

11. A plasmid vector comprising the nucleic acid of claim 8.

12. A mammalian cell into which a nucleic acid of claim 8 or a vector of one of claims 9-11 has been introduced.

13. A recombinant mammalian alkaline phosphatase polypeptide, wherein the polypeptide is capable of being expressed in a mammalian cell and of being detected in a mammal about 40 days after being first expressed in the cell of the mammal, wherein the polypeptide has the amino acid sequence of SEQ ID NO: 1 or 2.

14. The polypeptide of claim 13, wherein the polypeptide has a sequence with at least 95% amino acid identity to SEQ ID NO: 1 or 2.

15. The polypeptide of claim 13, wherein the polypeptide has a sequence with at least 90% amino acid identity to SEQ ID NO: 1 or 2.

16. The polypeptide of claim 13, wherein the polypeptide has a sequence with at least 80% amino acid identity to SEQ ID NO: 1 or 2.

17. A mammalian cell comprising the alkaline phosphatase polypeptide of claim 13.

18. A mammalian cell comprising the alkaline phosphatase polypeptide of claim 14.

19. A mammalian cell comprising the alkaline phosphatase polypeptide of claim 15.

20. A mammalian cell comprising the alkaline phosphatase polypeptide of claim 16.

21. A mouse comprising a cell of one of claims 17-20.

22. A macaque comprising a cell of one of claims 17-20.

## Patentansprüche

1. Isolierte Nucleinsäure, die eine Nucleotidsequenz umfasst, die eine alkalische Säuger-Phosphataseaktivität mit den Aminosäuresequenzen der SEQ.-ID. Nr. 1 oder 2 codiert, wobei die Nucleinsäure kein Polypeptid mit der gleichen Sequenz von Aminosäuren, die unter SEQ.-ID. Nr. 13 gelistet sind, codiert.

2. Expressionsvektor, der die Nucleinsäure nach Anspruch 1 umfasst.

3. Viraler Vektor, der die Nucleinsäure nach Anspruch 1 umfasst.

4. Plasmidvektor, der die Nucleinsäure nach Anspruch 1 umfasst.

5. Säugerzelle, in die eine Nucleinsäure nach Anspruch 1 oder einVektor nach einem der Ansprüche 2-4 *in vitro* eingebracht wurde.

6. Zelle nach Anspruch 5, die eine Mauszelle ist.

7. Zelle nach Anspruch 5, die eine menschliche Zelle ist.

8. Nucleinsäure mit einer Sequenz, die ein alkalische Säuger-Phosphataseaktivitätaufweisendes Polypeptid codiert, wobei das Polypeptid exprimiert und für einen Zeitraum von etwa 40 Tagen in einem Säuger nachgewiesen zu werden vermag und wobei die Nucleinsäure ein Polypeptid mit der Aminosäuresequenz der SEQ.-ID. Nr. 1 oder 2, jedoch nicht mit derjenigen der SEQ.-ID. Nr. 13 codiert.

9. Expressionsvektor, der die Nucleinsäure nach Anspruch 8 umfasst.

10. Viraler Vektor, der die Nucleinsäure nach Anspruch 8 umfasst.

11. Plasmidvektor, der die Nucleinsäure nach Anspruch 8 umfasst.

12. Säugerzelle, in die eine Nucleinsäure nach Anspruch 8 oder einVektor nach einem der Ansprüche 9-11 eingebracht wurde.

13. Rekombinantes alkalisches Säuger-Phosphatase-Polypeptid, wobei das Polypeptid in einer Säugerzelle exprimiert und in einem Säuger etwa 40 Tage nach der ersten Expression in der Zelle des Säugers nachgewiesen zu werden vermag, wobei das Polypeptid die Aminosäuresequenz der SEQ.-ID. Nr. 1 oder 2 aufweist.

14. Polypeptid nach Anspruch 13, wobei das Polypeptid eine Sequenz mit einer Aminosäure-Identität zur SEQ.-ID. Nr. 1 oder 2 von mindestens 95 % aufweist.

15. Polypeptid nach Anspruch 13, wobei das Polypeptid eine Sequenz mit einer Aminosäure-Identität zur SEQ.-ID. Nr. 1 oder 2 von mindestens 90 % aufweist,

16. Polypeptid nach Anspruch 13, wobei das Polypeptid eine Sequenz mit einer Aminosäure-Identität zur SEQ.-ID. Nr. 1 oder 2 von mindestens 80 % aufweist.

17. Säugerzelle, die das alkalische Phosphatase-Polypeptid nach Anspruch 13 umfasst.

18. Säugerzelle, die das alkalische Phosphatase-Polypeptid nach Anspruch 14 umfasst.

19. Säugerzelle, die das alkalische Phosphatase-Polypeptid nach Anspruch 15 umfasst.

20. Säugerzelle, die das alkalische Phosphatase-Polypeptid nach Anspruch 16 umfasst.

21. Maus, die eine Zelle nach einem der Ansprüche 17-20 umfasst.

22. Makak, der eine Zelle nach einem der Ansprüche 17-20 umfasst.

## Revendications

1. Acide nucléique isolé comprenant une séquence nucléotidique qui code pour une activité de phosphatase alcaline de mammifère avec les séquences d'acides aminés de SEQ ID NO : 1 ou 2, l'acide nucléique ne codant pas pour un polypeptide avec la même séquence d'acides aminés listée dans SEQ ID NO : 13.

2. Vecteur d'expression comprenant l'acide nucléique suivant la revendication 1.

3. Vecteur viral comprenant l'acide nucléique suivant la revendication 1.

4. Vecteur de type plasmide comprenant l'acide nucléique suivant la revendication 1.

5. Cellule de mammifère dans laquelle un acide nucléique suivant la revendication 1 ou un vecteur suivant l'une des revendications 2 à 4 a été introduit in vitro.

6. Cellule suivant la revendication 5, qui est une cellule de souris.

7. Cellule suivant la revendication 5, qui est une cellule humaine.

8. Acide nucléique comportant une séquence qui code pour un polypeptide possédant une activité de phosphatase alcaline de mammifère, le polypeptide étant capable d'être exprimé et détecté dans un mammifère pendant une période d'environ 40 jours, et l'acide nucléique codant pour un polypeptide avec la séquence d'acides aminés de SEQ ID NO : 1 ou 2, mais pas avec celle de SEQ ID NO : 13.

9. Vecteur d'expression comprenant l'acide nucléique suivant la revendication 8.

10. Vecteur viral comprenant l'acide nucléique suivant la revendication 8.

11. Vecteur de type plasmide comprenant l'acide nucléique suivant la revendication 8.

12. Cellule de mammifère dans laquelle un acide nucléique suivant la revendication 8 ou un vecteur suivant l'une des revendications 9 à 11 a été introduit.

13. Polypeptide de phosphatase alcaline de mammifère recombinant dans lequel le polypeptide est capable d'être exprimé dans une cellule de mammifère et d'être détecté dans un mammifère environ 40 jours après avoir été pour la première fois exprimé dans la cellule du mammifère, le polypeptide ayant la séquence d'acides aminés de SEQ ID NO : 1 ou 2.

14. Polypeptide suivant la revendication 13, dans lequel le polypeptide présente une séquence ayant un degré d'identité d'acides aminés d'au moins 95 % par rapport à SEQ ID NO : 1 ou 2.

15. Polypeptide suivant la revendication 13, dans lequel le polypeptide présente une séquence ayant un degré d'identité d'acides aminés d'au moins 90 % par rapport à SEQ ID NO : 1 ou 2.

16. Polypeptide suivant la revendication 13, dans lequel le polypeptide présente une séquence ayant un degré d'identité d'acides aminés d'au moins 80 % par rapport à SEQ ID NO : 1 ou 2.

17. Cellule de mammifère comprenant le polypeptide de phosphatase alcaline suivant la revendication 13.

18. Cellule de mammifère comprenant le polypeptide de phosphatase alcaline suivant la revendication 14.

19. Cellule de mammifère comprenant le polypeptide de phosphatase alcaline suivant la revendication 15.

20. Cellule de mammifère comprenant le polypeptide de phosphatase alcaline suivant la revendication 16.

21. Souris comprenant une cellule suivant l'une des revendications 17 à 20.

22. Macaque comprenant une cellule suivant l'une des revendications 17 à 20.


### SEQUENCE LISTING

<110> AVENTIS PHARMA SA
<120> ENGINEERED SECRETED ALKALINE PHOSPHATASE (SEAP) REPORTER GENES AND POLYPEPTIDES
<130> ENGINEERED SEAP
<140> GC010002
   <141> 2001-06-21
<160> 13
<170> PatentIn Ver. 2.1
<210> 1
   <211> 488
   <212> PRT
   <213> Mus musculus
<400> 1
   Ile Pro Val Glu Glu Glu Asn Pro Ala Phe Trp Asn Arg Lys Ala Ala
   1               5                   10                  15
   Glu Ala Leu Asp Ala Ala Lys Lys Leu Lys Pro Ile Gln Thr Ser Ala
               20                  25                  30
   Lys Asn Leu Val Ile Leu Met Gly Asp Gly Met Gly Val Ser Thr Val
           35                  40                  45
   Thr Ala Thr Arg Ile Leu Lys Gly Gln Gln Gln Gly His Leu Gly Pro
       50                  55                  60
   Glu Thr Gln Leu Ala Met Asp Arg Phe Pro His Met Ala Leu Ser Lys
   65                  70                  75                  80
   Thr Tyr Asn Thr Asp Lys Gln Ile Pro Asp Ser Ala Gly Thr Gly Thr
                   85                  90                  95
   Ala Phe Leu Cys Gly Val Lys Thr Asn Met Lys Val Ile Gly Leu Ser
               100                 105                 110
   Ala Ala Ala Arg Phe Asn Gln Cys Asn Thr Thr Trp Gly Asn Glu Val
           115                 120                 125
   Val Ser Val Met His Arg Ala Lys Lys Ala Gly Lys Ser Val Gly Val
       130                 135                 140
   Val Thr Thr Thr Ser Val Gln His Ala Ser Pro Ala Gly Thr Tyr Ala
   145                 150                 155                 160
   His Thr Val Asn Arg Gly Trp Tyr Ser Asp Ala Gln Met Pro Ala Ser
                   165                 170                 175
   Ala Leu Gln Asp Gly Cys Lys Asp Ile Ser Thr Gln Leu Ile Ser Asn
               180                 185                 190
   Met Asp Ile Asp Val Ile Leu Gly Gly Gly Arg Lys Phe Met Phe Pro
           195                 200                 205
   Lys Gly Thr Pro Asp Gln Glu Tyr Pro Thr Asp Thr Lys Gln Ala Gly
       210                 215                 220
   Thr Arg Leu Asp Gly Arg Asn Leu Val Gln Glu Trp Leu Ala Lys His
   225                 230                 235                 240
   Gln Gly Ala Arg Tyr Val Trp Asn Arg Ser Glu Leu Ile Gln Ala Ser
                   245                 250                 255
   Leu Asn Arg Ser Val Thr His Leu Met Gly Leu Phe Glu Pro Asn Asp
               260                 265                 270
   Met Lys Tyr Glu Ile His Arg Asp Pro Ala Gln Asp Pro Ser Leu Ala
           275                 280                 285
   Glu Met Thr Glu Val Ala Val Arg Met Leu Ser Arg Asn Pro Lys Gly
       290                 295                 300
   Phe Tyr Leu Phe Val Glu Gly Gly Arg Ile Asp His Gly His His Glu
   305                 310                 315                 320
   Thr Val Ala Tyr Arg Ala Leu Thr Glu Ala Val Met Phe Asp Ser Ala
                   325                 330                 335
   Val Asp Lys Ala Asp Lys Leu Thr Ser Glu Gln Asp Thr Met Ile Leu
               340                 345                 350
   Val Thr Ala Asp His Ser His Val Phe Ser Phe Gly Gly Tyr Thr Gln
           355                 360                 365
   Arg Gly Ala Ser Ile Phe Gly Leu Ala Pro Phe Lys Ala Glu Asp Gly
       370                 375                 380
   Lys Ser Phe Thr Ser Ile Leu Tyr Gly Asn Gly Pro Gly Tyr Lys Leu
   385                 390                 395                 400
   His Asn Gly Ala Arg Ala Asp Val Thr Glu Glu Glu Ser Ser Asn Pro
                   405                 410                 415
   Thr Tyr Gln Gln Gln Ala Ala Val Pro Leu Ser Ser Glu Thr His Ser
               420                 425                 430
   Gly Glu Asp Val Ala Ile Phe Ala Arg Gly Pro Gln Ala His Leu Val
           435                 440                 445
   His Gly Val Gln Glu Gln Asn Tyr Ile Ala His Val Met Ala Phe Ala
       450                 455                 460
   Ala Cys Leu Glu Pro Tyr Thr Asp Cys Gly Leu Ala Ser Pro Ala Gly
   465                 470                 475                 480
   Gln Ser Ser Ala Val Ser Pro Gly
                   485
<210> 2
   <211> 507
   <212> PRT
   <213> Mus musculus
<400> 2
   Met Trp Gly Ala Cys Leu Leu Leu Leu Gly Leu Ser Leu Gln Val Cys
   1 5 10 15
   Pro Ser Val Ile Pro Val Glu Glu Glu Asn Pro Ala Phe Trp Asn Arg
   20 25 30
   Lys Ala Ala Glu Ala Leu Asp Ala Ala Lys Lys Leu Lys Pro Ile Gln
   35 40 45
   Thr Ser Ala Lys Asn Leu Val Ile Leu Met Gly Asp Gly Met Gly Val
   50 55 60
   Ser Thr Val Thr Ala Thr Arg Ile Leu Lys Gly Gln Gln Gln Gly His
   65 70 75 80
   Leu Gly Pro Glu Thr Gln Leu Ala Met Asp Arg Phe Pro His Met Ala
   85 90 95
   Leu Ser Lys Thr Tyr Asn Thr Asp Lys Gln Ile Pro Asp Ser Ala Gly
               100                 105                 110
   Thr Gly Thr Ala Phe Leu Cys Gly Val Lys Thr Asn Met Lys Val Ile
           115                 120                 125
   Gly Leu Ser Ala Ala Ala Arg Phe Asn Gln Cys Asn Thr Thr Trp Gly
       130                 135                 140
   Asn Glu Val Val Ser Val Met His Arg Ala Lys Lys Ala Gly Lys Ser
   145                 150                 155                 160
   Val Gly Val Val Thr Thr Thr Ser Val Gln His Ala Ser Pro Ala Gly
                   165                 170                 175
   Thr Tyr Ala His Thr Val Asn Arg Gly Trp Tyr Ser Asp Ala Gln Met
               180                 185                 190
   Pro Ala Ser Ala Leu Gln Asp Gly Cys Lys Asp Ile Ser Thr Gln Leu
           195                 200                 205
   Ile Ser Asn Met Asp Ile Asp Val Ile Leu Gly Gly Gly Arg Lys Phe
       210                 215                 220
   Met Phe Pro Lys Gly Thr Pro Asp Gln Glu Tyr Pro Thr Asp Thr Lys
   225                 230                 235                 240
   Gln Ala Gly Thr Arg Leu Asp Gly Arg Asn Leu Val Gln Glu Trp Leu
                   245                 250                 255
   Ala Lys His Gln Gly Ala Arg Tyr Val Trp Asn Arg Ser Glu Leu Ile
               260                 265                 270
   Gln Ala Ser Leu Asn Arg Ser Val Thr His Leu Met Gly Leu Phe Glu
           275                 280                 285
   Pro Asn Asp Met Lys Tyr Glu Ile His Arg Asp Pro Ala Gln Asp Pro
       290                 295                 300
   Ser Leu Ala Glu Met Thr Glu Val Ala Val Arg Met Leu Ser Arg Asn
   305                 310                 315                 320
   Pro Lys Gly Phe Tyr Leu Phe Val Glu Gly Gly Arg Ile Asp His Gly
                   325                 330                 335
   His His Glu Thr Val Ala Tyr Arg Ala Leu Thr Glu Ala Val Met Phe
               340                 345                 350
   Asp Ser Ala Val Asp Lys Ala Asp Lys Leu Thr Ser Glu Gln Asp Thr
           355                 360                 365
   Met Ile Leu Val Thr Ala Asp His Ser His Val Phe Ser Phe Gly Gly
       370                 375                 380
   Tyr Thr Gln Arg Gly Ala Ser Ile Phe Gly Leu Ala Pro Phe Lys Ala
   385                 390                 395                 400
   Glu Asp Gly Lys Ser Phe Thr Ser Ile Leu Tyr Gly Asn Gly Pro Gly
                   405                 410                 415
   Tyr Lys Leu His Asn Gly Ala Arg Ala Asp Val Thr Glu Glu Glu Ser
               420                 425                 430
   Ser Asn Pro Thr Tyr Gln Gln Gln Ala Ala Val Pro Leu Ser Ser Glu
           435                 440                 445
   Thr His Ser Gly Glu Asp Val Ala Ile Phe Ala Arg Gly Pro Gln Ala
       450                 455                 460
   His Leu Val His Gly Val Gln Glu Gln Asn Tyr Ile Ala His Val Met
   465 470 475 480
   Ala Phe Ala Ala Cys Leu Glu Pro Tyr Thr Asp Cys Gly Leu Ala Ser
   485 490 495
   Pro Ala Gly Gln Ser Ser Ala Val Ser Pro Gly
   500 505
<210> 3
   <211> 19
   <212> PRT
   <213> Mus musculus
<400> 3
   Met Trp Gly Ala Cys Leu Leu Leu Leu Gly Leu Ser Leu Gln Val Cys
   1 5 10 15
   Pro Ser Val
<210> 4
   <211> 41
   <212> DNA
   <213> Artificial Sequence
<220>
   <223> Description of Artificial Sequence: OLIGONUCLEOTIDE
<400> 4
   gtataagctt gccaccatgt ggggagcctg cttgctgctg c 41
<210> 5
   <211> 38
   <212> DNA
   <213> Artificial Sequence
<220>
   <223> Description of Artificial Sequence: OLIGONUCLEOTIDE
<400> 5
   gttttctaga tcagcccggg ctcactgcac tgctctgg 38
<210> 6
   <211> 35
   <212> DNA
   <213> Artificial Sequence
<220>
   <223> Description of Artificial Sequence: OLIGONUCLEOTIDE
<400> 6
   actctctaga tcagggttca gccgccgcca tcagc 35
<210> 7
   <211> 35
   <212> DNA
   <213> Artificial Sequence
<220>
   <223> Description of Artificial Sequence: OLIGONUCLEOTIDE
<400> 7
   cgcgaagctt gccaccatgt ggggagcctg cttgc 35
<210> 8
   <211> 36
   <212> DNA
   <213> Artificial Sequence
<220>
   <223> Description of Artificial Sequence: OLIGONUCLEOTIDE
<400> 8
   ctcctctaga ctatcagccc gggctcactg cactgc 36
<210> 9
   <211> 24
   <212> PRT
   <213> Homo sapiens
<400> 9
   Met Ala Leu Trp Met Arg Leu Leu Pro Leu Leu Ala Leu Leu Ala Leu
   1 5 10 15
   Trp Gly Pro Asp Pro Ala Ala Ala
   20
<210> 10
   <211> 24
   <212> PRT
   <213> Mus musculus
<400> 10
   Met Ala Leu Leu Val His Phe Leu Pro Leu Leu Ala Leu Leu Ala Leu
   1               5                   10                  15
   Trp Glu Pro Lys Pro Thr Gln Ala
               20
<210> 11
   <211> 20
   <212> PRT
   <213> Mus musculus
<400> 11
   Met Glu Arg His Trp Ile Phe Leu Phe Leu Phe Ser Val Thr Ala Gly
   1               5                   10                  15
   Val His Ser Gln
               20
<210> 12
   <211> 20
   <212> PRT
   <213> RAT
<400> 12
<210> 13
   <211> 529
   <212> PRT
   <213> Mus musculus
<400> 13
   Met Trp Gly Ala Cys Leu Leu Leu Leu Gly Leu Ser Leu Gln Val Cys
   1               5                   10                  15
   Pro Ser Val Ile Pro Val Glu Glu Glu Asn Pro Ala Phe Trp Asn Arg
               20                  25                  30
   Lys Ala Ala Glu Ala Leu Asp Ala Ala Lys Lys Leu Lys Pro Ile Gln
           35                  40                  45
   Thr Ser Ala Lys Asn Leu Val Ile Leu Met Gly Asp Gly Met Gly Val
       50                  55                  60
   Ser Thr Val Thr Ala Thr Arg Ile Leu Lys Gly Gln Gln Gln Gly Leu
   65                  70                  75                      80
   Ser Gly Pro Glu Thr Gln Leu Ala Met Asp Arg Phe Pro His Met Ala
                   85                  90                  95
   Leu Ser Lys Thr Tyr Asn Thr Asp Lys Gln Ile Pro Asp Ser Ala Gly
               100                 105                 110
   Thr Gly Thr Ala Phe Leu Cys Gly Val Lys Thr Asn Met Lys Val Ile
           115                 120                 125
   Gly Leu Ser Ala Ala Ala Arg Phe Asn Gln Cys Asn Thr Thr Trp Gly
       130                 135                 140
   Asn Glu Val Val Ser Val Met His Arg Ala Lys Lys Ala Gly Lys Ser
   145                 150                 155                     160
   Val Gly Val Val Thr Thr Thr Ser Val Gln His Ala Ser Pro Ala Gly
                   165                 170                 175
   Thr Tyr Ala His Thr Val Asn Arg Gly Trp Tyr Ser Asp Ala Gln Met
               180                 185                 190
   Pro Ala Ser Ala Leu Gln Asp Gly Cys Lys Asp Ile Ser Thr Gln Leu
           195                 200                 205
   Ile Ser Asn Met Asp Ile Asp Val Ile Leu Gly Gly Gly Arg Lys Phe
       210                 215                 220
   Met Phe Pro Lys Gly Thr Pro Asp Gln Glu Tyr Pro Thr Asp Thr Lys
   225                 230                 235                     240
   Gln Ala Gly Thr Arg Leu Asp Gly Arg Asn Leu Val Gln Glu Trp Leu
                   245                 250                 255
   Ala Lys His Gln Gly Ala Arg Tyr Val Trp Asn Arg Ser Glu Leu Ile
               260                 265                 270
   Gln Ala Ser Leu Asn Arg Ser Val Thr His Leu Met Gly Leu Phe Glu
           275                 280                 285
   Pro Asn Asp Met Lys Tyr Glu Ile His Arg Asp Pro Ala Gln Asp Pro
       290                 295                 300
   Ser Leu Ala Glu Met Thr Glu Val Ala Val Arg Met Leu Ser Arg Asn
   305                      310                      315                      320
   Pro Lys Gly Phe Tyr Leu Phe Val Glu Gly Gly Arg Ile Asp His Gly
                   325                     330                     335
   His His Glu Thr Val Ala Tyr Arg Ala Leu Thr Glu Ala Val Met Phe
               340                     345                     350
   Asp Ser Ala Val Asp Lys Ala Asp Ile Arg Thr Ser Glu Gln Asp Thr
           355                     360                     365
   Met Ile Leu Val Thr Ala Asp His Ser His Val Phe Ser Phe Gly Gly
       370                     375                     380
   Tyr Thr Gln Arg Gly Ala Ser Ile Phe Gly Leu Ala Pro Phe Lys Ala
   385                     390                     395                     400
   Glu Asp Gly Lys Ser Phe Thr Ser Ile Leu Tyr Gly Asn Gly Pro Gly
                   405                     410                     415
   Tyr Lys Leu His Asn Gly Ala Arg Ala Asp Val Thr Glu Glu Glu Ser
               420                     425                     430
   Ser Asn Pro Thr Tyr Gln Gln Gln Ala Cys Val Pro Leu Ser Ser Glu
           435                     440                     445
   Thr His Ser Gly Glu Asp Val Ala Ile Phe Ala Arg Gly Pro Gln Ala
       450                     455                     460
   His Leu Val His Gly Val Gln Glu Gln Asn Tyr Ile Ala His Val Met
   465                     470                     475                     480
   Ala Phe Ala Ala Cys Leu Glu Pro Tyr Thr Asp Cys Gly Leu Ala Ser
                   485                     490                     495
   Pro Ala Gly Gln Ser Ser Ala Val Ser Pro Gly Tyr Met Ser Thr Leu
               500                     505                     510
   Leu Cys Leu Leu Ala Gly Lys Met Leu Met Leu Met Ala Ala Ala Glu
           515                     520                     525
   Pro